(19) Europäisches Patentamt
European Patent Office
Office européen des brevets

(11) Veröffentlichungsnummer: **0 173 252**
**B1**

(12) # EUROPÄISCHE PATENTSCHRIFT

(45) Veröffentlichungstag der Patentschrift: **26.07.89**

(51) Int. Cl.⁴: **C 08 G 18/79**, C 08 G 18/16, C 07 D 251/34, C 07 D 229/00

(21) Anmeldenummer: **85110545.2**

(22) Anmeldetag: **22.08.85**

(54) **Verfahren zur Herstellung von oligomeren Polyisocyanaten und ihre Verwendung bei der Herstellung von Polyurethankunststoffen.**

(30) Priorität: **31.08.84 DE 3432081**

(43) Veröffentlichungstag der Anmeldung: **05.03.86 Patentblatt 86/10**

(45) Bekanntmachung des Hinweises auf die Patenterteilung: **26.07.89 Patentblatt 89/30**

(84) Benannte Vertragsstaaten: **BE DE FR GB IT NL**

(56) Entgegenhaltungen: **GB-A-1 173 018**

(73) Patentinhaber: **BAYER AG, D-5090 Leverkusen 1 Bayerwerk (DE)**

(72) Erfinder: **Richter, Roland, Dr., Roggendorfstrasse 55, D-5000 Köln 80 (DE)**
Erfinder: **Müller, Hanns Peter, Dr., Im Kerberich 6, D-5068 Odenthal (DE)**
Erfinder: **Kubitza, Werner, Dipl.-Ing., Eduard-Spranger-Strasse 22, D-5090 Leverkusen 3 (DE)**
Erfinder: **Engbert, Theodor, Dr., Goethestrasse 57c, D-4047 Dormagen (DE)**
Erfinder: **Mennicken, Gerhard, Dr., Am Wasserturm 16, D-5090 Leverkusen 3 (DE)**

**EP 0 173 252 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von oligomeren, Uretdion- und/oder Isocyanurat-Struktureinheiten aufweisenden Polyisocyanaten durch Di- und/oder Trimerisierung eines Teils der Isocyanatgruppen von organischen Polyisocyanaten unter Verwendung von tertiären Phosphinen oder von peralkylierten Phosphorigsäuretriamiden als Dimerisierungs- und/oder Trimerisierungskatalysator und Abbruch der Di- und/oder Trimerisierungsreaktion durch Zugabe eines Katalysatorengifts, wobei als Katalysatorengift Sulfonylisocyanate, gegebenenfalls in Kombination mit organischen Säurechloriden, verwendet werden, sowie die Verwendung der Verfahrensprodukte bei der Herstellung von Polyurethankunststoffen.

Die Herstellung von Isocyanato-uretdionen oder von Isocyanato-isocyanuraten oder von deren Mischungen durch Di- und/oder Trimerisierung eines Teils der Isocyanatgruppen von organischen Polyisocyanaten unter Verwendung von aliphatischen, araliphatischen oder gemischt aliphatisch-aromatischen tert. Phosphinen bzw. von peralkylierten Phosphorigsäuretriamiden als Katalysatoren ist bereits bekannt. Die Frage, ob bei dieser Umsetzung vorwiegend Uretdiongruppen aufweisende Dimerisierungsprodukte oder vorwiegend Isocyanuratgruppen aufweisende Trimerisierungsprodukte entstehen, hängt im wesentlichen von dem Grad der Umsetzung und der Temperaturführung ab (vgl. A. Farkas und G.A. Mills, Adv. Catal. 13 [1962] Seite 393ff.).

Im allgemeinen kann gesagt werden, dass die Umsetzung mit steigender Temperatur und steigender Reaktionsdauer über die Vorstufe der Uretdione hinausgehend zu den Isocyanuratgruppen aufweisenden Trimeren mit vorwiegend einem Isocyanuratring und schliesslich zu Isocyanuratgruppen aufweisenden Polyisocyanaten führt, die neben Monoisocyanuraten auch mehr als einen Isocyanuratring aufweisende Homologe aufweisen. Sowohl die vorwiegend reinen Dimerisierungsprodukte als auch deren Gemische mit den Trimerisierungsprodukten als auch die vorwiegend reinen Trimerisierungsprodukte stellen jeweils wertvolle Ausgangsmaterialien für die Herstellung von Polyurethankunststoffen dar, wobei man bei der Herstellung der oligomeren Polysocyanate den Dimerisierungs- bzw. Trimerisierungsgrad jeweils dem angestrebten Verwendungszweck anpasst. In jedem Fall ist es jedoch für eine reproduzierbare grosstechnische Produktion unerlässlich, die Dimerisierungs- und/oder Trimerisierungsreaktion genau und schnell an einem vorbestimmten Punkt zu beenden.

Um ein derartiges scharfes Abstoppen der Reaktion beim gewünschten Umsetzungsgrad zu erreichen, werden häufig der Reaktionsmischung Verbindungen zugesetzt, die die Wirksamkeit des Katalysators aufheben sollen.

Nach der DE-OS 1 670 667 und DE-OS 1 670 720 sind dies vorzugsweise Alkylierungsmittel wie Dimethylsulfat, Methyliodid oder Toluolsulfonsäureester und Acylierungsmittel wie Carbonsäurechloride und Carbamidsäurechloride. Diese Mittel sind jedoch mit dem Nachteil behaftet, dass sie die Wirkung des Katalysators nicht augenblicklich und vollständig beenden, sondern dass im allgemeinen ein Nachheizen erforderlich ist (so wird in der DE-OS 1 670 720 eine Temperaturerhöhung auf 80°C vorgeschlagen), wodurch ein scharf kontrollierter Abbruch der Polymerisationsreaktion unmöglich ist.

Nach der Lehre der DE-AS 1 954 093 eignet sich Schwefel zur Desaktivierung der tert. Phosphin-Katalysatoren. Schwefel hat zwar den Vorzug, spontan und schnell abzustoppen, jedoch ist das entstehende Phosphinsulfid im allgemeinen eine leichtflüchtige Verbindung, welche bei der destillativen Entfernung des nicht umgesetzten Ausgangspolyisocyanats aus dem Reaktionsgemisch mit diesem abdestillieren kann und somit das Destillat, welches normalerweise recyclisiert wird, mit zunehmendem Masse (Kumulierung) verunreinigt.

Bei den Verfahren der US-PS 3 290 288, der DE-OS 3 030 513 und der DE-OS 3 227 779 werden bevorzugt peralkylierte Phosphorigsäuretriamide wie z. B. Tris-(dimethylamino)-phosphin als Katalysatoren verwendet, wobei gleichzeitig vorgeschlagen wird, das Reaktionsprodukt bei Erreichen des gewünschten Umsetzungsgrades ohne Desaktivierung des Katalysators einer Destillation zu unterwerfen. Obwohl die genannten Katalysatoren vor allem die Dimerisierung, nicht jedoch die Trimerisierung beschleunigen, besteht bei dieser Verfahrensweise dennoch die Möglichkeit, dass während der erhöhten Destillationstemperatur aus den Uretdionen und den überschüssigen Ausgangspolyisocyanaten unkontrollierbare Mengen an Trimerisaten entstehen, weswegen in DE-OS 3 030 513 und DE-OS 3 227 779 ausschliesslich der Einsatz von sterisch gehinderten (cyclo-)aliphatischen Ausgangsdiisocyanaten empfohlen wird, so dass davon ausgegangen werden muss, dass das Verfahren dieser Vorveröffentlichungen nur bei Verwendung spezieller Ausgangsdiisocyanate in einigermassen reproduzierbarer Weise die Herstellung von dimeren Diisocyanaten gestattet. Im übrigen muss darauf hingewiesen werden, dass das Destillat, welches den aktiven Katalysator enthält, nicht über einen längeren Zeitraum zwischengelagert werden kann, bevor es erneut eingesetzt wird, da es in einem solchen Falle durchpolymerisieren und somit unbrauchbar würde.

Es war daher die der Erfindung zugrundeliegende Aufgabe, ein neues Verfahren zur Dimerisierung- und/oder Trimerisierung von organischen Polyisocyanaten unter Verwendung der genannten Katalysatoren des Standes der Technik zur Verfügung zu stellen, welches eine einwandfreie und unverzügliche Abstoppung der Reaktion beim jeweils erwünschten Umsetzungsgrad gestattet, ohne mit den Nachteilen des genannten Standes der Technik behaftet zu sein.

Diese Aufgabe konnte mit dem nachstehend näher beschriebenen erfindungsgemässen Verfahren gelöst werden.

Gegenstand der Erfindung ist ein Verfahren zur Herstellung von oligomeren Polyisocyanaten durch Di- und/oder Trimerisierung eines Teils der Isocyanatgruppen von organischen Polyisocyanaten in Gegenwart von tert. Phosphinen oder eines peralkylierten Phosphorigsäuretriamids als Katalysator und Abbruch der Di- und/oder Trimerisierungsreaktion beim jeweils gewünschten Oligomerisierungsgrad durch Zugabe eines Katalysatorengifts, dadurch gekennzeichnet, dass man als Katalysatorengift ein Sulfonylisocyanat verwendet.

Gegenstand der Erfindung ist auch die Verwendung der nach diesem Verfahren erhaltenen, gegebenenfalls im Gemisch mit nicht umgesetztem Ausgangspolyisocyanat oder gegebenenfalls in mit Blockierungsmitteln für Isocyanatgruppen blockierter Form vorliegenden oligomeren Polyisocyanaten als Polyisocyanatkomponente bei der Herstellung von Polyurethankunststoffen.

Die Eignung der erfindungsgemäss als Katalysatorgift eingesetzten Sulfonylisocyanate zur Desaktivierung der beim erfindungsgemässen Verfahren eingesetzten Katalysatoren, insbesondere der tert. Phosphine, war überraschend, da man aus der einschlägigen Literatur entnehmen konnte, dass es sich bei den entsprechenden Addukten um sehr labile Verbindungen handelt. So reagiert Triphenylphosphin nur mit dem hochreaktiven Fluorsulfonylisocyanat (H. Hoffmann, H. Förster und G. Tor-Poghossian, Mh. Chem., Bd. 100 [1969], Seite 312) und dem Sulfonyldiisocyanat (R. Appel und H. Rittersbacher, Chem. Ber. 97 [1964], Seite 852 ff.) zu einem definierten Addukt, während die Umsetzung mit Chlorsulfonylisocyanat uneinheitlich verläuft. Vom Tosylisocyanat ist nur die Umsetzung mit tert.-Butylmethylphenylphosphin bei −78°C zu einem 1:1-Addukt bekannt, welches bei Raumtemperatur instabil ist (C.R. Hall und D.J.H. Smith, J.C.S. Perkin II [1977], Seite 1381 bis 1382).

Beim erfindungsgemässen Verfahren können beliebige organische Polyisocyanate mit aliphatisch, cycloaliphatisch, aromatisch und/oder araliphatisch gebundenen Isocyanatgruppen eingesetzt werden. Vorzugsweise werden organische Diisocyanate mit aliphatisch, cycloaliphatisch oder aromatisch gebundenen Isocyanatgruppen des Molekulargewichtsbereichs 168 bis 300 verwendet. Beliebige Gemische derartiger Poly- bzw. Diisocyanate können selbstverständlich auch eingesetzt werden.

Zu den als Ausgangsmaterialien geeigneten Polyisocyanaten gehören beispielsweise solche der Formel

$$Q(NCO)_n$$

in welcher

n für eine ganze Zahl von 2 bis 4, vorzugsweise für 2 steht und

Q für einen aliphatischen Kohlenwasserstoffrest mit 2 bis 18, vorzugsweise 6 bis 10 Kohlenstoffatomen, einen cyclialiphatischen Kohlenwasserstoffrest mit 4 bis 15, vorzugsweise 5 bis 10 Kohlenstoffatomen, einen aromatischen Kohlenwasserstoffrest mit 6 bis 15, vorzugsweise 6 bis 13 Kohlenstoffatomen oder einen araliphatischen Kohlenwasserstoffrest mit 8 bis 15, vorzugsweise 8 bis 13 Kohlenstoffatomen steht.

Beispiele derartiger Polyisocyanate sind Ethylendiisocyanat, 1,4-Tetramethylendiisocyanat, Hexamethylendiisocyanat, 1,12-Dodecamethylendiisocyanat, Cyclobutan-1,3-diisocyanat, Cyclohexan-1,3- und -1,4-diisocyanat, 1-Isocyanato-3,3,5-trimethyl-5-isocyanato-methylcyclohexan (Isophorondiisocyanat), Hexahydro-1,3- und/oder -1,4-phenylendiisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, 1-Methyl-1-isocyanato-4-(1-isocyanato-but-3-yl)-cyclohexan, 1-Methyl-1-isocyanato-4-isocyanatomethyl-cyclohexan, 2,6-Diisocyanato-n-hexansäuremethylester, («Lysin-methylester-diisocyanat»), Triphenylmethan-4,4',4''-triisocyanat oder Polyphenyl-polymethylenpolyisocyanate, wie sie durch Anilin-Formaldehyd-Kondensation und anschliessende Phosgenierung erhalten werden.

Zu den bevorzugten Ausgangsmaterialien für das erfindungsgemässe Verfahren gehören Hexamethylendiisocyanat, Isophorondiisocyanat, Perhydro-2,4'- und -4,4'-diisocyanatodiphenylmethan, 2,4-Diisocyanatotoluol, dessen technische Gemische mit bis zu 35 Gew.-%, bezogen auf Gesamtgewicht, an 2,6-Diisocyanatotoluol, 4,4'-Diisocyanatodiphenylmethan, dessen Gemische mit 2,4'-Diisocyanatodiphenylmethan mit einem Gehalt an 2,4'-Isomeren von bis zu 60 Gew.-%, bezogen auf Gesamtgemisch, sowie beliebige Gemische der genannten Diisocyanate, insbesondere beliebige Gemische von aromatischen Diisocyanaten der genannten Art mit (cyclo-)aliphatischen Diisocyanaten der genannten Art.

Als Katalysatoren werden beim erfindungsgemässen Verfahren tert. Phosphine oder peralkylierte Phosphorigsäuretriamide eingesetzt. Selbstverständlich können auch Gemische aus tert.-Phosphinen und peralkylierten Phosphorigsäuretriamiden verwendet werden, obwohl dies weniger bevorzugt ist. Geeignete tert. Phosphine sind insbesondere aliphatische, araliphatische oder gemischt aliphatisch-aromatische Phosphine des Molekulargewichtsbereichs 76 bis 500, d.h. dieser Definition entsprechende Verbindungen der allgemeinen Formel

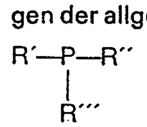

in welcher

R', R'' und R''' für gleiche oder verschiedene Reste stehen und Alkylgruppen mit 1 bis 10, vorzugsweise 2 bis 8 Kohlenstoffatomen, Aralkylgruppen mit 7 bis 10, vorzugsweise 7 Kohlenstoffatomen oder Arylgruppen mit 6 bis 10, vorzugsweise 6 Kohlenstoffatomen bedeuten, mit der Massgabe, dass höchstens einer der Reste für eine Arylgruppe und vorzugsweise mindestens einer der Reste für eine Alkylgruppe steht, oder in welcher zwei dieser Reste aliphatischer Natur sind und, miteinander verknüpft, zusammen mit dem Phosphoratom einen Phosphor als Heteroatom enthaltenden Ring mit 4 bis 6 Ringgliedern bilden, in welchem Falle der dritte der genanten Reste für eine Alkylgruppe mit 1 bis 4 Kohlenstoffatomen steht.

Beispiele geeigneter tert. Phosphine sind Triethylphosphin, Dibutylethylphosphin, Tri-n-propylphosphin, Tri-iso-propylphosphin, Tri-tert.-butylphosphin, Tribenzylphosphin, Benzyldimethylphosphin, Dimethylphenylphosphin, Tri-n-butylphosphin, Tri-iso-butylphosphin, Triamylphosphin, Trioctylphosphin oder Butyl-phosphacyclopentan. Tri-(n-butyl)-phosphin ist besonders gut als Katalysator für das erfindungsgemässe Verfahren geeignet.

Als Katalysatoren geeignete peralkylierte Phosphorigsäuretriamide sind beliebige organische Verbindungen der allgemeinen Formel

$$P(NR_2)_3$$

in welcher die einzelnen Reste R für gleiche oder verschiedene, vorzugsweise gleiche Alkylreste mit 1 bis 10, vorzugsweise 1 bis 4 Kohlenstoffatomen, Aralkylreste mit 7 bis 10, vorzugsweise 7 Kohlenstoffatomen oder Cycloalkylreste mit 4 bis 10, vorzugsweise 6 Kohlenstoffatomen stehen. Aus dieser Definition der Reste R wird ersichtlich, dass der Begriff «peralkylierte» im Rahmen der Erfindung breit dahingehend zu interpretieren ist, dass nicht nur echte Alkylreste sondern auch Cycloalkyl- und Aralkylreste als mögliche Substituenten des Stickstoffatoms in Betracht kommen. Vorzugsweise handelt es sich jedoch bei den erfindungsgemäss als Katalysatoren einzusetzenden peralkylierten Phosphorigsäuretriamiden um solche der genannten allgemeinen Formel, für welche alle Reste R für Alkylreste mit 1 bis 4 Kohlenstoffatomen, vorzugsweise für Methylreste stehen. Das permethylierte Phosphorigsäuretriamid, d.h. Tris-(dimethylamino)-phosphin ist der bevorzugte beim erfindungsgemässen Verfahren zum Einsatz gelangende Katalysator aus der Klasse der Phosphorigsäuretriamide.

Die Katalysatoren werden bei der Durchführung des erfindungsgemässen Verfahrens im allgemeinen in einer Menge von 0,01 bis 2 Gew.-%, bezogen auf die Menge des Ausgangspolyisocyanats, eingesetzt, wobei im Falle der Verwendung von vorwiegend aromatischen Polyisocyanaten als Ausgangspolyisocyanat vorzugsweise 0,01 bis 0,1 Gew.-% und im Falle der Verwendung von vorwiegend (cyclo-)aliphatischen Polyisocyanaten als Ausgangspolyisocyanat vorzugsweise 0,1 bis 1 Gew.-% des Katalysators, jeweils bezogen auf die Menge des Ausgangspolyisocyanats, zum Einsatz gelangt.

Das erfindungsgemässe Verfahren kann in Gegenwart oder Abwesenheit von Lösungsmitteln durchgeführt werden. Bei der Verwendung von Lösungsmitteln wird vorzugsweise eine etwas höhere Menge an Katalysator innerhalb der genannten Bereiche verwendet. Geeignete inerte organische Lösungsmittel sind z.B. Dioxan, Ester wie Methylacetat, Ethylacetat, Butylacetat und Methylglykolacetat; Ketone wie Aceton, Methylethylketon und Cyclohexanon; aromatische, aliphatische oder auch chlorierte Kohlenwasserstoffe mit Ausnahme von Tetrachlorkohlenstoff.

Erfindungsgemäss geeignete Sulfonylisocyanate sind beliebige anorganische oder organische Verbindungen, die mindestens eine Struktureinheit der Formel

$$-SO_2-NCO$$

aufweisen. Vorzugsweise werden organische Sulfonylisocyanate, besonders bevorzugt solche mit aromatisch gebundenen Isocyanatosulfonylresten eingesetzt. Verfahren zur Herstellung von organischen Sulfonylisocyanaten der erfindungsgemäss geeigneten Art, sowie ihr chemisches Verhalten werden zusammenfassend dargestellt von H. Ulrich in Chem. Rev. 65, Seite 369-376, 1965. Des weiteren ist die Herstellung von Arylsulfonylisocyanaten beschrieben in den US-PS 2 666 787 und 3 484 466. Erfindungsgemäss können sowohl aliphatische, cycloaliphatische als auch aromatische Mono- oder Polysulfonylisocyanate eingesetzt werden. Als Beispiel seien genannt:

Methylsulfonylisocyanat, Butylsulfonylisocyanat, Cyclohexylsulfonylisocyanat, Perfluoroctylsulfonylisocyanat, Phenylsulfonylisocyanat, p-Toluolsulfonylisocyanat, Benzylsulfonylisocyanat, p-Chlorphenylsulfonylisocyanat, m-Nitrophenylsulfonylisocyanat, 2,5-Dimethylphenylsulfonylisocyanat, p-Fluorphenylsulfonylisocyanat, 2,5-Dichlorphenylsulfonylisocyanat, 3,4-Dichlorphenylsulfonylisocyanat, p-Bromphenylsulfonylisocyanat, p-Methoxyphenylsulfonylisocyanat, p-Nitrophenylsulfonylisocyanat und o-Nitrophenylsulfonylisocyanat; m-Phenylendisulfonyldiisocyanat, p-Phenylendisulfonyldiisocyanat, 4-Methyl-m-phenylendisulfonyldiisocyanat, 2-Chlor-p-phenylendisulfonyldiisocyanat, 5-Chlor-m-phenylendisulfonyldiisocyanat, 1,5-Naphthylendisulfonyldiisocyanat, 3-Nitro-p-phenylendisulfonyldiisocyanat, 4-Methoxy-m-phenylendisulfonyldiisocyanat, 2,5-Furandiyl-bis(methylensulfonyl)-diisocyanat, 4,4'-Bisphenylendisulfonyldiisocyanat, 2,2'-Dichlor-4,4'-biphenylylendisulfonyldiisocyanat, 3,3'-Dimethoxy-4,4'-biphenylylendisulfonyldiisocyanat, (Methylendi-p-phenylen)disulfonyldiisocyanat, (Methylendi-3,3'-dimethoxy-p-phenylen)-disulfonyldiisocyanat, (Methylen-di-3,3'-dimethyl-p-phenylen)disulfonyldiisocya-

nat und 2-Methyl-p-phenylendisulfonyldiisocyanat; des weiteren Sulfonylisocyanate mit freien NCO-Gruppen wie m-Isocyanatophenylsulfonylisocyanat, p-Isocyanatophenylsulfonylisocyanat, 3-Isocyanato-p-tolylsulfonylisocyanat, 5-Isocyanato-o-tolylsulfonylisocyanat, 3-Isocyanato-4-methoxyphenylsulfonylisocyanat, 4-Isocyanato-3-chlorphenylsulfonylisocyanat, 4'-Isocyanato-4-biphenylylsulfonylisocyanat, 4'-Isocyanato-2,2'-dichloro-4-biphenylylsulfonylisocyanat, 4'-Isocyanato-3,3'-dimethoxy-4-biphenylylsulfonylisocyanat, α-(p-Isocyanatophenyl)-p-tolylsulfonylisocyanat, α-(4-Isocyanato-3-methoxyphenyl)-2-methoxy-p-tolylsulfonylisocyanat, α-(4-Isocyanato-m-tolyl)-2,4-xylylsulfonylisocyanat und 5-Isocyanato-1-naphthylsulfonylisocyanat; oder mit freien Isothiocyanat-Gruppen wie p-Isothiocyanatophenylsulfonylisocyanat, m-Isothiocyanatophenylsulfonylisocyanat, 3-Isothiocyanato-4-methoxyphenylsulfonylisocyanat und 4-Isothiocyanato-3-methylphenylsulfonylisocyanat.

Bevorzugte Verwendung finden Sulfonylisocyanate, deren $-SO_2-NCO$-Gruppe direkt an einen aromatischen Rest gebunden ist; ganz bevorzugt sind Phenylsulfonylisocyanat, p-Chlorphenylsulfonylisocyanat und p-Toluolsulfonylisocyanat (Tosylisocyanat).

Neben den beispielhaft genannten organischen Sulfonylisocyanaten kommen erfindungsgemäss auch anorganische Sulfonylisocyanate wie z.B. Chlorsulfonylisocyanat oder Sulfonyldiisocyanat, deren Herstellung beispielsweise in DE-PS 928 896 bzw. in DE-PS 1 152 023 beschrieben ist, in Betracht. Geeignet sind auch Oxy-sulfonylisocyanate wie z.B. Trimethylsilyloxysulfonylisocyanat.

Im allgemeinen ist es zur Abstoppung der erfindungsgemässen Umsetzung ausreichend, dem Reaktionsgemisch eine dem Katalysator äquivalente Menge eines der beispielhaft genannten Sulfonylisocyanate zuzusetzen (molares Verhältnis von $-SO_2-NCO$-Gruppen zu Phosphoratomen des Katalysators $\geqq 1$), d.h. in dem allgemein üblichen Fall der Verwendung von monofunktionellen Phosphinen und monofunktionellen Sulfonylisocyanaten werden letztere in mindestens äquimolaren Mengen, bezogen auf den Phosphinkatalysator zugesetzt. Will man jedoch nicht reagiertes monomeres Ausgangsmaterial nach Beendigung der Reaktion z.B. durch Destillation entfernen, so sind mindestens doppelt äquivalente Mengen, d.h. mindestens zwei Mol Sulfonylisocyanatgruppen pro Mol Phosphor des Katalysators erforderlich, um ein vollständig phosphorfreies Destillat zu erhalten. Dies gilt insbesondere im Falle der Verwendung der bevorzugten Ausgangsmaterialien wie z.B. Hexamethylendiisocyanat und/oder 2,4- bzw. 2,6-Diisocyanatotoluol, bei Verwendung des bevorzugten Katalysators Tri-n-butylphosphin und bei Verwendung des besonders bevorzugten Katalysatorengifts p-Toluolsulfonylisocyanat (Tosylisocyanat). Dies ist insofern überraschend, als das Additionsprodukt aus Tri-n-butylphosphin und Tosylisocyanat, welches in einem inerten Milieu hergestellt wird, unabhängig von den molaren Verhältnissen ein 1:1-Addukt ist.

Verwendet man dagegen Chlorsulfonylisocyanat oder ein Oxy-sulfonylisocyanat wie das Trimethylsiloxysulfonylisocyanat als Abstopper, so ist bereits die einfach molare Menge ausreichend, um ein lagerstabiles (NCO-Konstanz)Destillat zu erhalten.

Eine weitere Möglichkeit, die Verfahrensprodukte durch Destillation von nicht umgesetztem, überschüssigem Ausgangspolyisocyanat zu befreien, und gleichzeitig ein Phosphin-freies Destillat zu erhalten, besteht darin, neben dem erfindungswesentlichen Sulfonylisocyanat ein organisches Säurechlorid als weiteres Katalysatorengift einzusetzen. Hierbei werden die erfindungswesentlichen Sulfonylisocyanate in, bezogen auf den Phosphor des Phosphins, mindestens äquivalenten Mengen zusammen mit enem organischen Säurechlorid eingesetzt, wobei die Gesamtmenge der beiden Arten von Katalysatorengift in, bezogen auf den Phosphor des Katalysators, mindestens doppelt äquivalenten Mengen zum Einsatz gelangen (Molverhältnis von P-Atomen des Katalysators zu Sulfonylisocyanat- und Säurechloridgruppen $\leqq 0,5$). Die Zugabe des Säurechlorids kann hierbei zusammen mit der Zugabe des Sulfonylisocyanats oder im Anschluss daran erfolgen.

Geeignete organische Säurechloride sind insbesondere Carbonsäurechloride wie z.B. Benzoylchlorid, iso-Phthalsäuredichlorid, Carbamidsäurechloride wie z.B. Phenylcarbamidsäurechlorid, Toluyl-2,4-biscarbamidsäurechlorid, Hexycarbamidsäurechlorid oder, allerdings weniger bevorzugt, Sulfonsäurechloride wie z.B. Benzolsulfonsäurechlorid oder p-Toluolsulfonsäurechlorid.

Im Falle der Verwendung von Sulfonylisocyanaten, deren Siedepunkt im Bereich bzw. unterhalb des Siedepunkts des monomeren Ausgangsdiisocyanats liegt, besteht die Möglichkeit, dass bei der Herstellung von monomerenfreien Verfahrensprodukten durch Abdestillieren von überschüssigem, nicht umgesetztem Ausgangspolyisocyanat nach Abstoppung der Reaktion Sulfonylisocyanat im Destillat vorliegt. Diese unerwünschte Möglichkeit ist insbesondere dann gegeben, wenn zur Abstoppung eine höher als äquivalente Menge an Sulfonylisocyanat oder neben einer äquivalenten Menge an Sulfonylisocyanat zusätzlich noch ein organisches Säurechlorid verwendet wird. In beiden Fällen kann das im Destillat vorliegende Sulfonylisocyanat in einfacher Weise dadurch vernichtet werden, dass man dem Destillat eine geringe, d.h. dem destillierten Sulfonylisocyanat mindestens äquivalente Menge einer Substanz einverleibt, die mit dem hochreaktiven Sulfonylisocyanat zu inerten, nicht flüchtigen Produkten abreagiert. Bei der nachfolgenden Wiederverwendung des Destillats in einem neuen Raktionsansatz verbleibt das inerte Umsetzungsprodukt im Reaktionsprodukt (Destillationsrückstand), ohne dessen Eigenschaften nachteilhaft zu beeinflussen. Im Falle der Verwendung von äquivalenten Mengen eines Sulfonylisocyanats und der zusätzlichen Verwendung einer mindestens äquivalenten Menge eines organischen Säurechlorids kann auch so vorgegangen werden, dass man dem Reaktionsgemisch nach der Abstoppung der Reaktion und vor der destillativen Entfernung des überschüssigen Ausgangspolyisocyanats einen derartigen «Fänger» für Sulfonylisocyanate hinzugibt. Auch in diesem Falle weist das Destillat kein freies Sulfonylisocyanat mehr auf. Eine Erklärung für diese überraschende Beobachtung steht nicht zur Verfügung.

Geeignete Fänger für Sulfonylisocyanate, insbesondere für das erfindungsgemäss bevorzugte Tosylisocyanat, sind z.B. ein- oder mehrwertige Alkohole wie tert. Butanol, Tetraethylenglykol, 2-Ethyl-1,3-hexandiol, 3-Methyl-1,5-pentandiol, 2,5-Dimethyl-2,5-hexandiol oder 2,5-Hexandiol; tert. Amine wie z.B. N,N-Dimethylbenzylamin oder N,N-Dimethyl-cyclohexylamin; Aldehyde wie Benzaldehyd; Sulfoxide wie Tetramethylensulfoxid oder Dimethylsulfoxid oder Silanole wie Trimethylsilanol.

Die Durchführung des erfindungsgemässen Verfahrens geschieht in an sich bekannter Weise, wie z.B. in DE-AS 1 670 667, DE-AS 1 954 093 oder DE-OS 3 227 779 beschrieben. Im allgemeinen wird das Ausgangspolyisocyanat in Gegenwart des Katalysators bei 0 bis 100 °C, vorzugsweise 20 bis 60 °C gehalten, bis ein Umsetzungsgrad von 5 bis 70, vorzugsweise 20 bis 60% erreicht ist. In diesem Zusammenhang ist unter «Umsetzungsgrad» der Prozentsatz der Isocyanatgruppen des Ausgangspolyisocyanats zu verstehen, der während der erfindungsgemässen Umsetzung unter Di- und/oder Trimerisierung abreagiert. Der Umsetzungsgrad kann leicht über die Abnahme des NCO-Gehalts des Reaktionsgemischs kontrolliert werden. Ob beim erfindungsgemässen Verfahren vorwiegend Uretdion-modifizierte Polyisocyanate (Dimere) oder vorwiegend Isocyanuratmodifizierte Polyisocyanate (Trimere) entstehen, hängt sowohl vom Umsetzungsgrad als auch von der gewählten Reaktionstemperatur ab. Im allgemeinen handelt es sich bei den erfindungsgemäss erhältlichen Umsetzungsprodukten um Gemische aus Dimeren und Trimeren mit, von den genannten Parametern abhängig, schwankendem Gehalt der genannten Modifizierungsprodukte. Falls solche Ausgangspolyisocyante eingesetzt werden, deren Uretdione in überschüssigem Ausgangspolyisocyanat unlöslich sind, kann die gegebenenfalls erwünschte, zumindest teilweise Weiterreaktion der Dimeren zu den Trimeren durch Mitverwendung einen Lösungsmittels der oben beispielhaft genannten Art, die ein Ausfällen des Dimeren verhindern, erreicht werden.

Zur Abstoppung der Reaktion wird dem Reaktionsgemisch bei dem gewünschten NCO-Gehalt des Katalysatorgift zugesetzt. Die Zugabe des Sulfonylisocyanats bewirkt einen sofortigen Abbruch der Reaktion auch im Temperaturbereich von 20° bis 60 °C; ein Nachheizen ist nicht erforderlich. Nicht reagiertes monomeres Ausgangsisocyanat kann nach Beendigung der Reaktion und gegebenfalls einer kurzzeitigen Nachrührphase (10 bis 60 Min.) durch beliebige Trennoperationen wie z.B. Destillation, insbesondere Dünnschichtdestillation, oder Extraktion entfernt werden und nach Vernichtung von evtl. vorhandenem Sulfonylisocyanat durch einen der zuvor beispielhaft genannten Fänger zur erneuten Modifizierung ohne Beeinträchtigung der Aktivität wieder eingesetzt werden.

Neben der Wiederverwendbarkeit des Destillats weisen die erfindungsgemässen Produkte den Vorteil auf, eine hohe Lagerstabilität zu besitzen, was sich durch konstante Viskosität, und Farbe und einen konstanten NCO-Gehalt bemerkbar macht. Insbesondere besteht bei Verwendung der genannten Katalysatorgifte nicht die Gefahr, dass bei der Aufarbeitung die im Verlauf der Umsetzung aus tert. Phosphinen und Luftsauerstoff leicht entstehenden Phosphinoxide zur Nebenproduktbildung (Carbodiimidisierung) führen, da z.B. Tosylisocyanat mit Phosphinoxiden zu keinerlei katalytische Wirkung aufweisenden Phosphiniminen (unter $CO_2$-Abspaltung) abreagiert (vgl. C.R. Hall und D.J.H. Smith, J.C.S. Perkin II [1977], Seite 1373 ff.).

Die erfindungsgemäss erhältlichen Verfahrensprodukte stellen wertvolle Ausgangsmaterialien zur Herstellung von Polyurethankunststoffen, insbesondere zur Herstellung von Polyurethanlacken und Polyurethanklebstoffen dar. Sie können hierbei sowohl in von überschüssigem Ausgangspolyisocyanat weitgehend befreiter Form als auch in Form ihrer Lösungen in überschüssigem Ausgangspolyisocyanat als auch, gewünschtenfalls, in mit an sich bekannten Blockierungsmitteln blockierter Form in an sich bekannter Weise zum Einsatz gelangen.

Die in den nachfolgenden Beispielen genannten Teile beziehen sich auf Gewichtsteile und die Prozentangaben auf Gewichtsprozente.

Beispiel 1

(Vergleich verschiedener Katalysatorgifte mit dem erfindungsgemäss zu verwendenden Katalysatorgift)

In einem geeigneten Reaktionsgefäss werden 200 Gew.-Teile Hexamethylendiisocyanat mit

A: 1,0 Teilen Tri-n-butylphosphin
B: 1,0 Teilen Tri-n-butylphosphin und 0,95 Teilen p-Toluolsulfonsäuremethylester
C: 1,0 Teilen Tri-n-butylphosphin und 0,5 Teilen Isophthalsäurechlorid
D: 1,0 Teilen Tri-n-butylphosphin und 1,0 Teilen Dibutylphosphat
E: 1,0 Teilen Tri-n-butylphosphin und 0,75 Teilen 2-Ethyl-hexansäure
F: 1,0 Teilen Tri-n-butylphosphin und 0,16 Teilen elem. Schwefel
G: 1,0 Teilen Tri-n-butylphosphin und 0,98 Teilen p-Toluolsulfonylisocyanat (Tosylisocyanat)

vermischt und bei 20 °C unter $N_2$-Atmosphäre gerührt.

Die angegebenen Mengen der einzelnen Katalysatorengifte sind der Menge des Phosphins in allen Fällen äquivalent. Der Umsatzgrad wird durch NCO-Messung in den nachfolgend angegebenen Zeiträumen bestimmt:

| NCO-Gehalt nach: | 3 Stdn. | 6 Stdn. | 24 Stdn. |
|---|---|---|---|
| A: | 44,5% | 42,4% | 32,2% |
| B: | 45,2% | 44,4% | 41,4% |
| C: | 49,1% | 48,8% | 48,4% |
| D: | 49,5% | 48,5% | 42,2% |
| E: | 44,9% | 42,2% | 33,6% |
| F: | 49,9% | 49,9% | 49,9% |
| G: | 49,8% | 49,8% | 49,8% |

Das Beispiel zeigt, dass sowohl Alkylierungsmittel (B) und Acylierungsmittel (C) als auch saure Verbindungen (D,E) bei 20 °C keine ausreichend desaktivierende Wirkung aufweisen.

Beispiel 2

a) In einem geeigneten Reaktionsgefäss werden unter trockenem Stickstoff 1000 g (5,95 Mol) Hexamethylendiisocyanat vorgelegt und bei Raumtemperatur mit 2,0 g (0,01 Mol) Tri-n-butylphosphin verrührt. Nach 18 Stunden Rührzeit ist der NCO-Gehalt auf 40,5% abgefallen. Man stoppt die Reaktion durch Zugabe von 3,9 g (0,02 Mol) Tosylisocyanat ab, rührt 30 Min. nach und destilliert dann im Dünnschichtverdampfer bei 160 °C und 0,15 mbar monomeres Hexamethylendiisocyanat ab. Es werden 680 g farbloses Destillat und 320 g eines monomerenfreien (<0,5%), gelblichen Sumpfproduktes mit einem NCO-Gehalt von 22,7%, einem Gehalt am Einkern-Uretdion (Mol-Masse 336; gelchromatographische Bestimmung) von 39% und einer Viskosität von 110 mPas (25 °C) erhalten. Der NCO-Gehalt des Destillats ist nach einmonatiger Lagerung unverändert.

b) Das Destillat wird nach Abmischung mit 320 g (1,9 Mol) frischem Hexamethylendiisocyanat und Einrühren von 2,5 g (0,017 Mol) 2-Ethyl-1,3-hexandiol erneut unter den oben angegebenen Bedingungen polymerisiert und aufgearbeitet. Man erhält 315 g eines gelblichen Polyisocyanatharzes mit einem NCO-Gehalt von 22,2% und einer Viskosität von 108 mPas (25 °C) sowie 685 Teile farbloses Destillat, welches sich in analoger Weise recyclisieren lässt.

Beispiel 3 (Vergleich)

Identisch mit Beispiel 2, jedoch werden nach Erreichen des NCO-Gehaltes von 40,5% 0,32 g (0,01 Mol) elementarer Schwefel als Abstopper eingerührt. Nach analoger Aufarbeitung erhält man ein leicht gelbliches Destillat mit einem Gehalt an Tributylphosphinsulfid von 0,34%, der sich bei weiterer Recyclisierung entsprechend verdoppelt und zudem die Farbqualität der nachfolgenden Produkte negativ beeinflusst (dunkelrotgelbe Verfärbung).

Beispiel 4

Identisch mit Beispiel 2, jedoch werden nach Erreichen des NCO-Gehaltes von 40,5% nur 2,0 g (0,01 Mol) Tosylisocyanat und zusätzlich 1,0 g (0,005 Mol) Isophthalsäuredichlorid eingerührt und danach analog Beispiel 2 aufgearbeitet.

Der NCO-Gehalt dieses Destillats ist nach einmonatiger Lagerung ebenfalls unverändert, was anzeigt, dass auch bei dieser Verfahrensweise kein freies Phosphin im Destillat vorlag.

Die Produkte aus Beispiel 2, 3 und 4 werden bei 50 °C in verschlossenem Gebinde 1 Monat getempert. Alle drei Produkte weisen nach dieser Zeit einen NCO-Gehalt von 22,6 bis 22,7% auf und sind bezüglich Farbe und Viskosität unverändert.

Beispiel 5

In einem geeigneten Reaktionsgefäss werden unter trockenem Stickstoff 600 g (3,57 Mol) Hexamethylendiisocyanat vorgelegt und bei Raumtemperatur mit 1,5 g (0,007 Mol) Tri-n-butylphosphin verrührt. Nach 17 Stunden Rührzeit ist der NCO-Gehalt auf 41,1% abgefallen. Man stoppt die Reaktion durch Zugabe von 1,45 g (0,007 Mol) Trimethylsilyloxysulfonylisocyanat ab, rührt 30 Minuten nach und destilliert dann im Dünnschichtverdampfer bei 160 °C und 0,15 mbar monomeres Hexamethylendiisocyanat ab. Man erhält 416 g farbloses Destillat, dessen NCO-Gehalt bei Lagerung völlig konstant bleibt, und 184 g eines gelben Polyisocyanat-Harzes mit 22,5% NCO-Gehalt, weniger als 0,4% freiem Hexamethylendiisocyanat und einer Viskosität von 117 mPas (25 °C). Das Reaktionsprodukt bleibt bei Lagerung über 6 Monate in seinen Kenndaten unverändert.

b) Analog zu Beispiel 5a, jedoch mit 1,0 g (0,007 Mol) Chlorsulfonylisocyanat als Abstopper, erhält man ebenfalls ein lagerstabiles Destillat und ein Polyisocyanat-Harz wie in Beispiel 5a, dessen Farbe in diesem Fall aber dunkelgelb ist.

Beispiel 6

In einem geeigneten Reaktionsgefäss werden unter trockenem Stickstoff 400 g (2,38 Mol) Hexamethylendiisocyanat vorgelegt. Dann werden 4,0 g (0,025 Mol frisch destilliertes Hexamethylphosphorigsäuretriamid zugegeben und die Reaktionsmischung unter gutem Rühren auf 60 °C erhitzt. Nach 45 Minuten ist der NCO-Gehalt der Reaktionsmischung auf 40 °C abgefallen und man gibt 9,7 g (0,05 Mol) Tosylisocyanat hinzu. nach einer Nachrührphase bei 60 °C von 30 Minuten beträgt der NCO-Gehalt 39,4% und das Verhältnis von Uretdion zu Isocyanurat im IR-Spektrum ca. 35:65 (Bande bei 1770 $cm^{-1}$ zu Bande bei 1690 $cm^{-1}$). Nach 20tägiger Lagerung bei 50 °C ist der NCO-Gehalt unverändert und hinsichtlich Farbe und des Uretdion/Isocyanurat-Verhältnisses sind ebenfalls keine signifikanten Veränderungen feststellbar. Die Aufarbeitung des Reaktionsgemisches durch Dünnschichtdestillation bei 160 °C/0,15 mbar ergibt 152 g eines bräunlichgelben Rückstands mit einem NCO-Gehalt von 21,1% und ein schwach gelbstichiges Destillat (248 g) mit einem NCO-Gehalt von 49,6%, der sich bei Lagerung nicht verändert.

Beispiel 7

170 g (0,98 Mol) 2,4-Toluylendiisocyanat werden mit 330 g (1,96 Mol) Hexamethylendiisocyanat auf 60 °C geheizt und unter Inertgasatmosphäre durch Zugabe von 0,125 g (0,6 mmol) Tri-n-butylphosphin polymerisiert. Durch leichtes Kühlen wird die Temperatur während der Reaktionsphase auf ca. 60 °C gehalten. Wenn nach ungefähr 4,5 Stunden ein NCO-Gehalt von 36% erreicht ist, stoppt man die Reaktion durch Zugabe von 0,25 g (1,3 mmol) Tosylisocyanat ab und rührt 30 Minuten bei 60 °C bis 40 °C nach, wobei nicht weiter geheizt werden muss. Anschliessend wird nicht umgesetztes Monomerengemisch in einem Dünnschichter (Umlauftemperatur 190 °C, Vakuum: 0,15 mbar) abdestilliert. Man erhält 180 g eines brüchigen gelben Festharzes mit einem NCO-Gehalt von 19,6% und 292 g farbloses Monomerengemisch als Destillat, dessen NCO-Gehalt (49,4%) konstant bleibt. Das Festharz besteht im wesentlichen aus Isocyanurat-Einheiten (Aliphatenanteil ca. 40%).

Beispiel 8

A) Addukt aus Tosylisocyanat und tert. Phosphin

In 50 ml abs. Hexan werden 3,0 g (14,9 mmol) Tri-n-butylphosphin gelöst und mit 2,93 g (14,9 mmol) Tosylisocyanat verrührt. Es scheidet sich ein weisses kristallines Festprodukt ab, welches unter Feuchtigkeitsausschluss isoliert und getrocknet wird.

Ausbeute: 5,0 g (84% der Theorie); Schmelzpunkt: 140 °C.

Struktur:

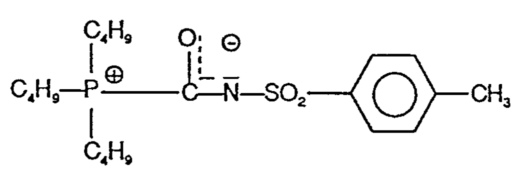

Analytische Daten:

IR: $V_{max.}$ (Nujol) 1620 $cm^{-1}$, 1440 $cm^{-1}$; $^{31}p$ (81 MHz): $\delta = 19,57$ ppm (rel. zu ext. $H_3PO_4$); $^{13}C$ (50,3 MHz): $\delta = 162,28$ ppm d, $^1H_{C,P} = 103,6$ Hz (weitere Signale unterhalb 141 ppm)

$C_{20}H_{34}NO_3PS$ (399) Ber.: S 8,0 N 3,51
Gef.: S 8,5 N 3,5

B) 500 g (2,98 Mol) Hexamethylendiisocyanat werden unter trockenem Stickstoff mit 2,0 g (0,005 Mol) Addukt aus Tosylisocyanat und Tributylphosphin (A), entsprechend 0,2% Tributylphosphin, 17 Stunden bei Raumtemperatur verrührt. Der NCO-Gehalt beträgt danach 49,3%. Die Lösung wird im Dünnschichtverdampfer bei 160 °C und 0,15 mbar destilliert. Man erhält 498 g farbloses Destillat mit einem NCO-Gehalt von 49,8%. Nach 18 Tagen Lagerung bei Raumtemperatur ist dieser Wert auf 45,5% abgesunken, was andeutet, dass unter den Destillationsbedingungen Phosphin zurückgebildet wird.

C) Analog zu B) werden 500 g (2,98 Mol) Hexamethylendiisocyanat mit 2,0 g (0,005 Mol) Addukt A und 1,0 g (0,005 Mol) Tosylisocyanat 17 Stunden bei Raumtemperatur gerührt. Der NCO-Gehalt beträgt danach 49,8%. Die Lösung wird wie unter B) angegeben destilliert. Man erhält 496 g farbloses Destillat mit einem NCO-Gehalt von 49,7%. Dieser Wert ist nach 18 Tagen Lagerung bei Raumtemperatur unverändert.

## Patentansprüche

1. Verfahren zur Herstellung von oligomeren Polyisocyanaten durch Di- und/oder Trimerisierung eines Teils der Isocyanatgruppen von organischen Polyisocyanaten in Gegenwart von tert. Phosphinen oder eines peralkylierten Phosphorigsäuretriamids als Katalysator und Abbruch der Di- und/oder Trimerisierungsreaktion beim jeweils gewünschten Oligomerisierungsgrad durch Zugabe eines Katalysatorengifts, dadurch gekennzeichnet, dass man als Katalysatorengift ein Sulfonylisocyanat verwendet.

2. Verfahren zur Herstellung von oligomeren Polyisocyanaten gemäss Anspruch 1, dadurch gekennzeichnet, dass man als Ausgangspolyisocyanate organische Diisocyanate des Molekulargewichtsbereichs 168 bis 300 mit aliphatisch, cycloaliphatisch und/oder aromatisch gebundenen Isocyanatgruppen verwendet.

3. Verfahren gemäss Anspruch 1 und 2, dadurch gekennzeichnet, dass man die Dimerisierungs- und/oder Trimerisierungsreaktion innerhalb des Temperaturbereichs von 0 °C bis 100 °C unter Dimerisierung und/oder Trimerisierung von 5 bis 70% der in den Ausgangspolyisocyanaten vorliegenden Isocyanatgruppen durchführt und die Reaktion bei Erreichen dieses Umsetzungsgrades durch Zugabe des Katalysatorengifts abbricht.

4. Verfahren gemäss Anspruch 1 bis 3, dadurch gekennzeichnet, dass man das als Katalysatorengift eingesetzte Sulfonylisocyanat in einer mindestens äquivalenten Menge, bezogen auf den Katalysator, verwendet.

5. Verfahren gemäss Anspruch 1 bis 4, dadurch gekennzeichnet, dass man das als Katalysatorgift eingesetzte Sulfonylisocyanat in einer mindestens doppelt äquivalenten Menge, bezogen auf den Katalysator, verwendet.

6. Verfahren gemäss Anspruch 1 bis 4, dadurch gekennzeichnet, dass man das als Katalysatorgift eingesetzte Sulfonylisocyanat in einer mindestens äquivalenten Menge, bezogen auf den Katalysator, zusammen mit mindestens einer solchen Menge eines organischen Säurechlorids einsetzt, dass für jedes Mol an Phosphoratomen des Katalysators insgesamt mindestens 2 Mol an Sulfonylisocyanat- und Säurechloridgruppen zur

Verfügung stehen, wobei die Zugabe des Säurechlorids gleichzeitig oder im Anschluss an die Zugabe des Sulfonylisocyanats erfolgt.

7. Verfahren gemäss Anspruch 1 bis 6, dadurch gekennzeichnet, dass man als Sulfonylisocyanat ein aromatisches Sulfonylisocyanat verwendet.

8. Verfahren gemäss Anspruch 1 bis 7, dadurch gekennzeichnet, dass man als Sulfonylisocyanat Tosylisocyanat verwendet.

9. Verfahren gemäss Anspruch 1 bis 8, dadurch gekennzeichnet, dass man im Anschluss an die Abstoppung der Umsetzung das im Überschuss vorliegende, nicht umgesetzte Ausgangspolyisocyanat destillativ aus dem Reaktionsansatz entfernt.

10. Verwendung der gemäss Anspruch 1 bis 9 erhaltenen, gegebenenfalls im Gemisch mit nicht umgesetztem Ausgangspolyisocyanat oder gegebenenfalls in mit Blockierungsmitteln für Isocyanatgruppen blockierter Form vorliegenden oligomeren Polyisocyanaten als Polyisocyanatkomponente bei der Herstellung von Polyurethankunststoffen.

## Revendications

1. Procédé de préparation de polyisocyanates oligomères par dimérisation et/ou trimérisation d'une partie des groupes isocyanates de polyisocyanates organiques en présence de phosphines tertiaires ou d'un triamide peralkylé d'acide phosphoreux comme catalyseur et arrêt de la réaction de dimérisation et/ou de trimérisation pour chaque degré souhaité d'oligomérisation par addition d'un poison du catalyseur, procédé caractérisé en ce qu'on utilise un isocyanate de sulfonyle comme poison du catalyseur.

2. Procédé pour préparer des polyisocyanates oligomères selon la revendication 1, caractérisé en ce qu'on utilise, comme polyisocyanates de départ, des diisocyanates organiques dont le poids moléculaire se situe entre 168 et 300 et qui comportent des groupes isocyanates fixés sur des fragments aliphatiques, cycloaliphatiques et/ou aromatiques.

3. Procédé selon la revendication 1 et la revendication 2, caractérisé en ce qu'on conduit la réaction de dimérisation et/ou de trimérisation dans un intervalle de température allant de 0°C à 100°C, avec dimérisation et/ou trimérisation de 5 à 70% des groupes isocyanates présents dans les polyisocyanates de départ et, lorsque ce degré de réaction est atteint, on arrête la réaction par l'addition du poison de catalyseur.

4. Procédé selon l'une des revendications 1 à 3, caractérisé en ce qu'on utilise l'isocyanate de sulfonyle, servant de poison de catalyseur, en une quantité au moins equivalente par rapport au catalyseur.

5. Procédé selon l'une des revendications 1 à 4, caractérisé en ce qu'on utilise l'isocyanate de sulfonyle, servant de poison de catalyseur, en une quntité correspondant au moins au double de l'équivalent par rapport au catalyseur.

6. Procédé selon l'une des revendications 1 à 4, caractérisé en ce qu'on introduit l'isocyanate de sulfonyle, servant de poison du catalyseur , en une quantité au moins équivalente, par rapport au catalyseur, avec au moins une quantité d'un chlorure d'acide organique telle que pour chaque mole d'atome de phosphore du catalyseur, il y ait au total au moins 2 moles de groupe isocyanate de sulfonyle et de groupe chlorure d'acide disponibles, l'addition du chlorure d'acide étant réalisée en même temps que l'addition de l'isocyanate de sulfonyle ou après l'addition de l'isocyanate de sulfonyle.

7. Procédé selon l'une des revendications 1 à 6, caractérisé en ce qu'on utilise, comme isocyanate de sulfonyle, un isocyanate de sulfonyle aromatique.

8. Procédé selon l'une des revendications 1 à 7, caractérisé en ce qu'on utilise comme isocyanate de sulfonyle l'isocyanate de tosyle.

9. Procédé selon l'une des revendications 1 à 8, caractérisé en ce qu'après l'arrêt de la réaction, on élimine, par distillation de la charge de réaction, le polyisocyanate de départ présent en excès et qui n'a pas réagi.

10. Utilisation des polyisocyanates oligomères obtenus selon l'une des revendications 1 à 9, éventuellement en mélange avec du polyisocyanate de départ n'ayant pas réagi ou éventuellement sous forme bloquée à l'aide d'agents de blocage des groupes isocyanates, comme composant polyisocyanate dans la préparation de matières plastiques de type polyuréthanne.

## Claims

1. A process for the production of oligomeric polyisocyanates by dimerizing and/or trimerizing some of the isocyanate groups in organic polyisocyanates in the presence of tertiary phosphines or a peralkylated phosphorous acid triamide as catalyst and terminating the dimerization and/or trimerization reaction at the required degree of oligomerization by the addition of a catalyst poison, characterized in that a sulfonyl isocyanate is used as the catalyst poison.

2. A process for the production of oligomeric polyisocyanates as claimed in Claim 1, characterized in that the starting polyisocyanates used are organic diisocyanates having a molecular weight in the range from 168 to 300 and containing aliphatically, cycloaliphatically and/or aromatically bound isocyanate groups.

3. A process as claimed in Claims 1 and 2, characterized in that the dimerization and/or trimerization reaction is carried out at 0°C to 100°C until from 5 to 70% of the isocyanate groups present in the starting polyisocyanates have been dimerized and/or trimerized, the reaction being terminated by addition of the catalyst poison when that degree of conversion is reached.

4. A process as claimed in Claims 1 to 3, characterized in that the sulfonyl isocyanate used as catalyst poison is used in an at least equivalent quantity, based on the catalyst.

5. A process as claimed in Claims 1 to 4, characterized in that the sulfonyl isocyanate used as catalyst poison is used in at least twice the equivalent quantity, based on the catalyst.

6. A process as claimed in Claims 1 to 4, characterized in that the sulfonyl isocyanate used as catalyst poison is used in an at least equivalent quantity, based on the catalyst, together with at least such a quantity of an organic acid chloride that in all at least 2 moles of sulfonyl isocyanate and acid chloride groups are available for each mole of phosphorous atoms of the catalyst, the acid chloride being added at the same time as or after the sulfonyl isocyanate.

7. A process as claimed in Claims 1 to 6, characterized in that an aromatic sulfonyl isocyanate is used as the sulfonyl isocyanate.

8. A process as claimed in Claims 1 to 7, characterized in that tosyl isocyanate is used as the sulfonyl isocyanate.

9. A process as claimed in Claims 1 to 8, characterized in that, after the reaction has been terminated, the unreacted starting polyisocyanate present in excess is removed from the reaction mixture by distillation.

10. The use of the oligomeric polyisocanates obtained in accordance with Claims 1 to 9, optionally in admixture with unreacted starting polyisocyanate or optionally blocked by blocking agents for isocyanate groups, as polyisocyanate component in the production of polyurethane plastics.